# EUROPÄISCHE PATENTSCHRIFT

(11) **EP 1 077 980 B1**
(45) Veröffentlichungstag und Bekanntmachung des Hinweises auf die Patenterteilung: **19.03.2003**
(21) Anmeldenummer: 99926300.7
(22) Anmeldetag: 07.05.1999
(51) Int. Cl.: C07D 493/00

(54) **EPITHILONDERIVATE, VERFAHREN ZU DEREN HERSTELLUNG UND DEREN VERWENDUNG**
EPOTHILONE DERIVATIVES, A METHOD FOR THE PRODUCTION THEREOF, AND THEIR USE
DERIVES D'EPOTHILONE, LEUR PROCEDE DE PREPARATION ET LEUR UTILISATION

(30) Priorität: 08.05.1998 DE 19820599
(43) Veröffentlichungstag der Anmeldung: 28.02.2001
(73) Patentinhaber: Gesellschaft für Biotechnologische Forschung mbH (GBF), D-38124 Braunschweig (DE)
(72) Erfinder: HOEFLE, Gerhard, D-38124 Braunschweig (DE); LEIBOLD, Thomas, D-38124 Braunschweig (DE)
(74) Vertreter: Boeters, Hans Dietrich, Dr.
(86) Internationale Anmeldenummer: EP9903159
(87) Internationale Veröffentlichungsnummer: WO99058534

(56) Entgegenhaltungen:
- WO-A-93/10121
- DE-A- 19 542 986
- NICOLAOU K C ET AL: "DESIGNED EPOTHILONES: COMBINATORIAL SYNTHESIS, TUBULIN ASSEMBLY PROPERTIES, AND CYTOTOXIC ACTION AGAINST TAXOL.RESISTANT TUMOR CELLS" ANGEWANDTE CHEMIE. INTERNATIONAL EDITION, Bd. 36, Nr. 19, 1. Januar 1997 (1997-01-01), Seiten 2097-2103, XP002064441 ISSN: 0570-0833
- BALOG A ET AL: "TOTAL SYNTHESIS OF (-)-EPOTBILINE A" ANGEWANDTE CHEMIE. INTERNATIONAL EDITION, Bd. 35, Nr. 23/24, 1. Januar 1996 (1996-01-01), Seiten 2801-2803, XP002035359 ISSN: 0570-0833
- NICOLAOU, K. C. ET AL: "Total synthesis of oxazole- and cyclopropane-containing epothilone B analogs by the macrolactonization approach" CHEM.--EUR. J. (1997), 3(12), 1971-1986 , XP002121564
- NICOLAOU, K. C. ET AL: "Total synthesis of oxazole- and cyclopropane-containing epothilone A analogs by the olefin metathesis approach" CHEM.--EUR. J. (1997), 3(12), 1957-1970 , XP002121565
- TORRADO A ET AL: "GENERAL SYNTHESIS OF RETINOIDS AND AROTINOIDS VIA PALLADIUM -CATALYZED CROSS-COUPLING OF BORONIC ACIDS WITH ELECTROPHILES" SYNTHESIS,1. März 1995 (1995-03-01), Seiten 285-293, XP002057741 ISSN: 0039-7881
- PETASIS N A ET AL: "Mild conversion of alkenyl boronic acids to alkenyl halides with halosuccinimides" TETRAHEDRON LETTERS, Bd. 37, Nr. 5, 29. Januar 1996 (1996-01-29), Seite 567-570 XP004030301 ISSN: 0040-4039
- SCHUMMER, DIETMAR ET AL: "An improved preparation of tris(ethylenedioxyboryl)methane, a reagent for the homologation of aldehydes and ketones" TETRAHEDRON (1995), 51(41), 11219 -22 , XP002121566 in der Anmeldung erwähnt
- AKIRA SUZUKI: "organoborates in new synthetic reactions" ACCOUNTS OF CHEMICAL RESEARCH., Bd. 15, 1981, Seiten 178-184, XP002121567 WASHINGTON US in der Anmeldung erwähnt
- NICOLAOU, K. C. ET AL: "Total synthesis of epothilone E and analogs with modified side chains through the Stille coupling reaction" ANGEW. CHEM., INT. ED. (1998), 37(1/2), 84-87 , XP002121568 in der Anmeldung erwähnt

## Beschreibung

Die vorliegende Erfindung betrifft Epothilonderivate der nachfolgend darstellten allgemeinen Formeln 2 bis 6, Verfahren zu deren Herstellung sowie Arzneimittel und Pflanzenschutzmittel mit diesen Epothilonderivaten.

In DE 195 42 986 sind bereits Epothilon A und Epothilon B sowie deren 3-O- und/oder 7-O-geschützte Derivate, Epothilone mit einer C = C-Bindung in 2,3-Stellung (Formel 6) und Epothilone mit einer Keto-Gruppe in C16-Stellung (Formel 4) bekannt; vgl. auch WO 93/10 121.

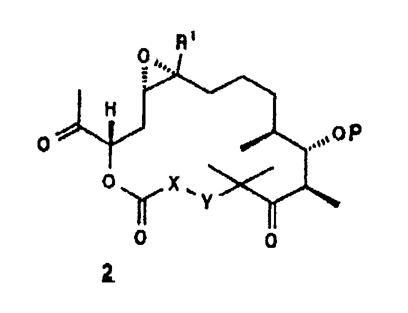

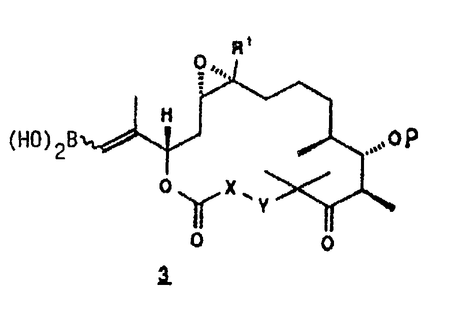

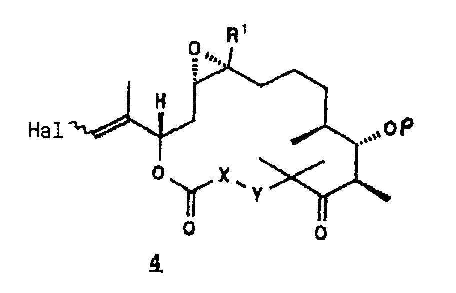

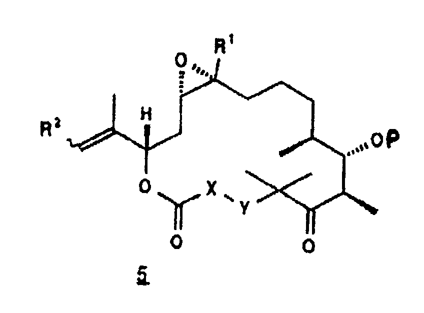

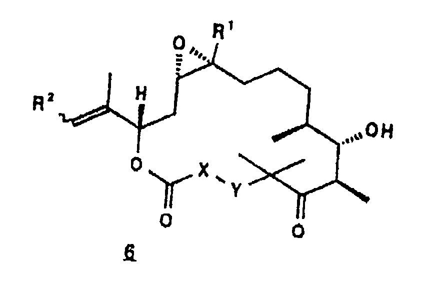

In den vorstehenden Formeln bedeutet:
R¹ = ein H-Atom oder eine C₁- bis C₈-Alkylgruppe, vorzugsweise eine C₁- bis C₆-Alkylgruppe, besonders bevorzugt eine C₁- bis C₄-Alkylgruppe, insbesondere eine Methyl-, Ethyl-, Propyl- oder Butylgruppe,
R² = ein monocyclischer Aromat, wie ein 5- oder 6-gliedriger Aromat (wie ein Phenylring), der durch ein, zwei, drei, vier oder fünf, insbesondere ein oder zwei Halogenatome und/oder OR⁴und/oder NR⁵R⁶-Gruppen und/oder Alkyl- und/oder Alkenyl- und/oder Alkinylgruppen in ortho- und/oder meta- und/oder para-Stellung substituiert sein kann, worin R⁴, R⁵ und R⁶ unabhängig voneinander dieselbe Bedeutung wie R¹ haben, aber von R¹ unabhängig sind, oder
R² = ein monocyclischer 5- oder 6-gliedriger Heteroaromat, der eines oder mehrere, insbesondere ein oder zwei O- und/oder N-und/oder S-Atome im Ring aufweisen kann und/oder OR⁴- und/oder NR⁵R⁶-Gruppen und/oder Alkyl- und/oder Alkenyl- und/oder Alkinylgruppen als Substituenten aufweisen kann, worin R⁴, R⁵ und R⁶ wie vorstehend defniert sind. Insbesondere werden bei der Definition von R² C₁-C₆-Alkyl-, bzw. C₂-C₆-Alkenyl- und -Alkinylgruppen, insbesondere C₁-C₄-Alkyl-, bzw. C₂-C₄-Alkenyl- und -Alkinylgruppen bevorzugt. Als Alkylgruppen werden besonders Methyl-, Ethyl-, Propyl- und Butylgruppen und als Heteroaromaten 6-gliedrige Heteroaromaten bevorzugt,
Hal = ein Halogenatom wie Br oder I,
X-Y = eine Gruppe der Formel -CH₂CH-OP oder -CH=CH-, und
P = eine Schutzgruppe wie TMS.

Die erfindungsgemäßen Verbindungen können wie folgt hergestellt werden:
Verbindungen der Formel (2) können dadurch hergestellt werden, daß Verbindungen der Formel (1) 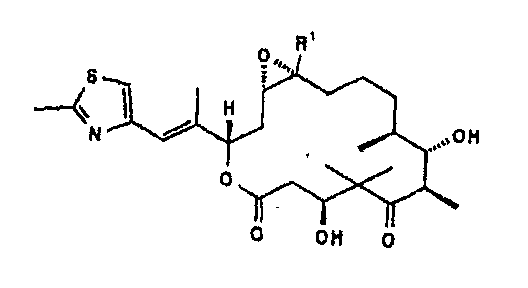 wie in der DE 195 42 986 beschrieben, umgesetzt werden, wobei die Reste wie vorstehend definiert sind. Insbesondere können dabei die folgenden Bedingungen (i), (iii) und gegebenenfalls (nach (i)) auch (ii) eingesetzt werden:
(i) (a) O₃ in einem Lösungsmittel wie CH₂Cl₂, und
   (b) reduktive Aufarbeitung, z.B. mit Me₂S;
(ii) (a) (CH₃CO)₂O, HCO₂H, NEt₃, DMAP;
   (b) DBU; und
   (c) MeOH, NH₃; und
(iii) Me₃SiCl, NEt₃.

Verbindungen der Formel (3) sind dadurch zugänglich, daß eine Verbindung der Formel (2) mit einer Verbindung der Formel HC[B(OR)₂]₃, wie Tris(ethylendioxyboryl)methan, umgesetzt wird. Dabei kann R eine wie vorstehend definierte Alkyl- oder Alkenylgruppe sein.

Bei der Umsetzung kommt gegebenenfalls eine starke Base, wie eine C₁-C₄ -Alkyl-Li-Verbindung (wie Butyllithium) oder eine Di-C₁-C₄-alkylamin-Li-Verbindung (wie eine Dimethylaminlithiumverbindung) zum Einsatz. Die Umsetzung wird in der Regel bei tiefen Temperaturen wie z.B. bei Temperaturen von weniger als von -30 °C, vorzugsweise bei Temperaturen von weniger als -50 °C, besonders bevorzugt bei Temperaturen von mindestens -78 °C durchgeführt. Weitere Reaktionsbedingungen können D. Schummer, G. Höfle in *Tetrahedron* **1995**, *51*, 11219 entnommen werden.

Beispielsweise wird eine Verbindung der Formel (2) mit Tris-(ethylendioxyboryl)methan und Butyllithium bei -78 °C zu einer Verbindung der Formel (3) umgesetzt.

Aus einer Verbindung der Formel (3) kann durch Umsetzung mit N-Jod- oder N-Bromsuccinimid, gegebenenfalls in einem polaren Lösungsmittel, wie Acetonitril, eine Verbindung der Formel (4) hergestellt werden. Weitere Reaktionsbedingungen können der folgenden Literaturstelle entnommen werden: N.A. Petasis, I.A. Zavialor, *Tetrahedron Lett*. **1996**, 37, 567.

Zur Herstellung einer Verbindung der Formel (5) kann eine Verbindung der Formel (3) im Rahmen einer Suzuki-Kopplung mit einer Verbindung der Formel R²-Z umgesetzt werden, wobei R² die vorstehend angegebenen Bedeutungen hat und Z ein Halogenatom oder eine Gruppe der Formel -OSO₂CF₃, -CH=CHI, -CH=CHOSO₂CF₃ sein kann. Insbesondere kann die Gruppe R²-Z die folgenden Strukturen aufweisen: 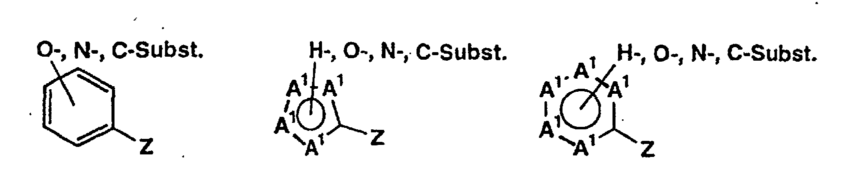 worin A¹ O, S, N oder C-Atome darstellt und die Substituenten O-, N- und C- den vorstehend beschriebenen Gruppen OR⁴-, NR⁵R⁶-, und Alkyl-, Alkenyl- und/oder Alkinylgruppen entsprechen.

Insbesondere werden C₁-C₆ Alkyl-, bzw. C₂-C₆ Alkenyl- und/oder Alkinylgruppen, insbesondere C₁-C₄ Alkyl-, bzw. C₂-C₄ Alkenylund/oder Alkinylgruppen als Substituenten "C" bevorzugt. Als Alkylgruppen werden besonders Methyl-, Ethyl-, Propyl- und Butylgruppen bevorzugt.

Alternativ kann eine Verbindung der Formel (5), dadurch hergestellt werden, daß eine Verbindung der Formel (4) durch eine Stille-Kupplung mit R²-SnR³₃ umgesetzt wird, wobei R² wie vorstehend definiert ist und R³ eine C₁- bis C₆-Alkylgruppe, vorzugsweise eine C₁- bis C₄-Alkylgruppe und besonders bevorzugt eine Methyl-, Ethyl-, Propyl- oder Butylgruppe ist. Außerdem kann die Verbindung R²-SnR³₃ eine der folgenden Strukturen aufweisen: 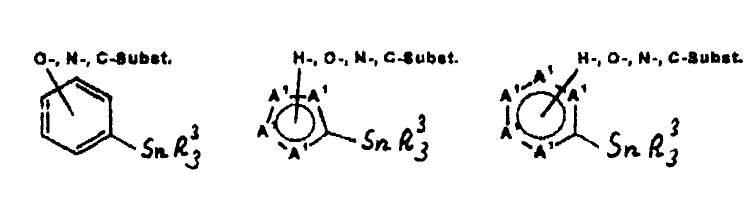 worin die Reste und Substituenten wie vorstehend definiert sind.

Erfindungsgemäß kann weiter eine Verbindung der Formel (6), dadurch hergestellt werden, daß von der Verbindung der Formel (5) die Schutzgruppe z.B. mit einer schwachen Säure, wie Zitronensäure oder Verbindungen wie TBAF, Pyridin x HF, entfernt wird. Gegebenenfalls kann dabei als Lösungsmittel ein Alkohol, wie Methanol, eingesetzt werden, wobei die Temperatur vorzugsweise auf Werte von z.B. 40 bis 60 °C, bevorzugt etwa 50 °C, eingestellt wird.

Insgesamt kann die Verbindung der Formel (6) durch die vorstehend beschriebenen Schritte (Epothilon A oder B → (2) → (3) → (4) → (5) → (6) oder Epothilon A oder B → (2) → (3) → (5) → (6)) hergestellt werden.

Weiter werden erfindungsgemäß Arzneimittel offenbart, die mindestens eine der Verbindungen (2), (3), (4), (5) oder (6) und gegebenenfalls übliche Träger, Verdünnungsmittel und Adjuvantien enthalten.

Insbesondere können derartige Verbindungen auch als Cytostatica und für den Pflanzenschutz in der Landwirtschaft und/oder Forstwirtschaft und/oder im Gartenbau eingesetzt werden, wobei sie gegebenenfalls zusammen mit einem oder mehreren üblichen Trägern, Adjuvantien und/oder Verdünnungsmitteln verwendet werden.

### Beispiele

### Synthese der Ketonderivate 2

Detaillierte Beschreibung siehe DE 195 42 986 A1.

### Synthese der Alkenylboronsäurederivate 3

(s. auch D. Schummer, G. Höfle *Tetrahedron* **1995**, *51,* 11219)

Typisches Beispiel (R¹ = H, X-Y = CH₂CHOTMS):

Tris(ethylendioxyboryl)methan (0,30 g, 1,5 mmol) wurde in CH₂Cl₂/THF (1:1; 4 ml) gelöst vorgelegt und unter Inertgas auf -78° C gekühlt. Bei dieser Temperatur wurde innerhalb von 10 min Buthyllithium (1,6 M-Lsg in Hexan; 0,73 ml, 1,2 mmol) zugetropft. Nach 2 h wurde Keton 2 (81 mg, 0,15 mmol) in CH₂Cl₂/THF (1:1; 2 ml) zugegeben, auf Raumtemperatur erwärmt und 17 h lang gerührt. Nach Versetzen mit MeOH (2 ml) wurde die klare Reaktionslösung mittels präparativer HPLC (Lichroprep RP-18, CH₃CN/H₂O 75 : 25) gereinigt. Es wurden 57 mg (65 %) Alkenylboronsäure 3 als E/Z-lsomerengemisch (6 : 4) erhalten.

Ausgewählte typische Daten: LC-MS (ESI-MS): 585 (M⁺+ H); ¹H-NMR: (300 MHz, CD₃OD): E-Isomer: 1,91(S, 3H), 5,16 (d, 1H, 10Hz), 5,49 (s, 1H), Z-Isomer; 1,85 (d, 3H, 1,1 Hz), 4,93 (s, 1H), 5,26 (d, 1H, 9,6 Hz)

### Synthese der Iodvinylderivate 4

(s. auch N.A. Petasis, I.A. Zavialor, *Tetrahedron Lett.* **1996,** 37, 567)

Typisches Beispiel (R¹ = H, X-Y = CH₂CHOTMS):

Bei Raumtemperatur wurde eine Lösung von Alkenylboronsäure 3 (12 mg, 21 µmol; *E*/*Z 9:1)* in CH₃CN (150 µl) unter Inertgas und Lichtausschluß mit N-lodsuccinimid (6,0 mg, 27 µmol) versetzt und 3 h gerührt. Nach Einengen wurde der Rückstand mittels präparativer Dünnschichtchromatographie (SiO₂, CH₂Cl₂/MeOH 95: 5) gereinigt. Es wurden 9 mg (66 %) des lodvinyl-Derivats 4 als E/Z-Isomerengemisch (9:1) isoliert.

Ausgewählte typische Daten: LC-MS (ESI-MS): 667 (M⁺ + H); ¹H-NMR: (300 MHz, CDCl₃): E-Isomer: 1,82 (d, 3H, 1,1 Hz), 5,36 (d, 1H, 11 Hz), 6,43 (s, 1H), Z-Isomer: 1,84 (d, 3H, 1,1 Hz), 5,54 (d, 1H, 10,5 Hz), 6,09 (s, 1H)

### Suzuki-Kupplung der Alkenylboronsäure 3

(s. auch A. Suzuki, *Acc. Chem. Res.* **1982**, *15*, 178; A. Torrado, S. Lopez, R. Alvarez, A. R. De Lera *Synthesis,* **1995***,* 285)

Typisches Beispiel (R¹ = H, X-Y = CH₂CHOTMS, R² = Ph):

Eine Lösung von Alkenylboronsäure 3 (12 mg, 21 µmol; E/Z 2: 8) und Thalliumethanolat (2M-Lsg in EtOH; 12 µl, 24 µmol) in THF (150 µl) wurde 15 min bei Raumtemperatur gerührt, dann eine Lösung von Phenyliodid (4,0 µl, 6,0 mg, 29 µmol) und Tetrakis(triphenylphosphino)palladium (7,1 mg, 6,2 µmol) in THF (150 µl) in 30 min zugetropft und erneut 30 min gerührt. Nach Reinigung mittels präparativer Dünnschichtchromatographie (SiO₂, CH₂Cl₂/Et₂O 95 : 5) wurde das phenylanaloge Epothilon 5 (10 mg, 79 %, E/Z 2 : 8) als farbloser Feststoff erhalten.

Ausgewählte typische Daten: LC-MS (ESI-MS) : 617 (M⁺ + H); ¹H-NMR: (300 MHz, CDCl₃): E-Isomer: 1,87 (d, 3H, 1,4 Hz), 5,35 (d, 1H, 10,7 Hz), 6,54 (s, 1H), Z-Isomer: 1,80 (d, 3H, 1,5 Hz), 5,61 (d, 1H, 10,2 Hz), 6,41(s, 1H)

### Stille-Kupplung der Iodvinylderivate 4

(s. auch K.C. Nicolaou, Y. He, F. Roschangar, N. P. King, D. Vourloumis, T. Li *Angew. Chem*. **1998**, *110*, (1/2), 89)

## Patentansprüche

1. Epothilonderivat der Formel (2) 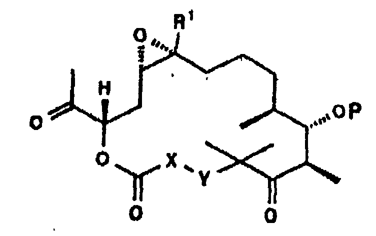 worin R¹ ein H-Atom oder eine C₁₋₈-Alkylgruppe, X-Y eine Gruppe der Formel -CH₂CH-OP oder -CH=CH-, und P eine Schutzgruppe ist, wobei X-Y als Gruppe der Formel -CH₂CH-OP ausgenommen ist, wenn R¹ ein H-Atom oder eine C₁₋₄-Alkylgruppe bedeutet.

2. Epothilonderivat der Formel (3) 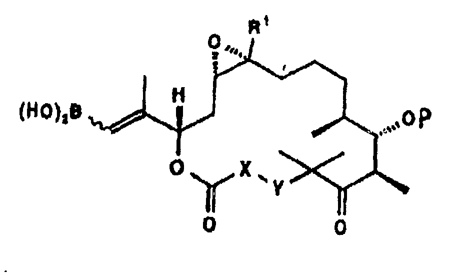 worin die Reste wie in Anspruch 1 definiert sind.

3. Epothilonderivat der Formel (4) 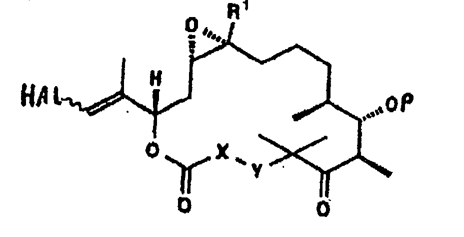 worin die Reste R¹, X-Y und P wie in Anspruch 1 definiert sind, und Hal ein Halogenatom wie Br oder J ist.

4. Epothilonderivat der Formel (5) 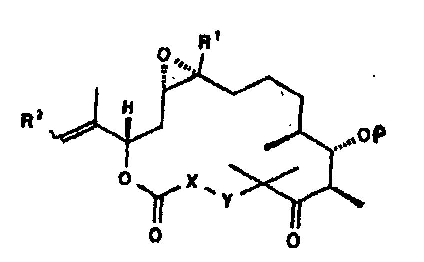 worin die Reste R¹, X-Y und P wie in Anspruch 1 definiert sind und
R² ein monocyclischer Aromat ist, der durch Halogenatome und/oder -OR⁴ und/oder -NR⁵R⁶ und/oder Alkyl-, Alkenyl- und/oder Alkinylgruppen in ortho- und/oder meta- und/oder para-Stellung substituiert sein kann, oder ein monocyclischer 5- oder 6-gliedriger Heteroaromat ist, der eines oder mehrere O- und/oder N- und/oder S-Atome im Ring aufweisen kann und/oder -OR⁴ und/oder -NR⁵R⁶ und/oder Alkyl-, Alkenyl- und/oder Alkinylgruppen als Substituenten aufweisen kann, wobei die Reste R⁴, R⁵ und R⁶ unabhängig voneinander wie R¹ in Anspruch 1 definiert sind, aber von R¹ unabhängig sind, wobei
(i) X-Y als Gruppe der Formel -CH=CH- ausgenommen ist, wenn R¹ ein H-Atom oder eine C₁₋₄-Alkylgruppe und R² einen mono-cyclischen Heteroaromaten mit einem N-Atom und einem S-Atom im Ring und mit einem C₁-Alkyl-Substituenten bedeuten und
(ii) X-Y als Gruppe der Formel -CH₂-CH-OP ausgenommen ist, wenn R¹ ein H-Atom oder eine C₁₋₄-Alkylgruppe und R² einen mono-cyclischen Heteroaromaten mit einem N-Atom und einem S-Atom im Ring und mit einem C₁-Alkyl-Substituenten bedeuten.

5. Epothilonderivat der Formel (6) 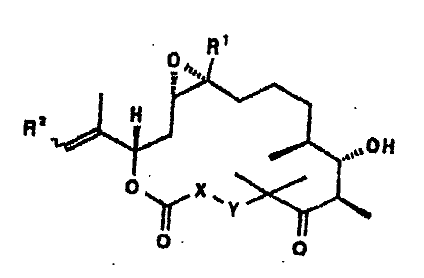 worin die Reste wie in Anspruch 4 definiert sind und, wenn X-Y eine Gruppe der Formel -CH₂CH-OP bedeutet, deren Schutzgruppe P entfernt ist, wobei
(i) X-Y als Gruppe der Formel -CH=CH- ausgenommen ist, wenn R¹ ein H-Atom oder eine C₁₋₄-Alkylgruppe und R² einen mono-cyclischen Heteroaromaten mit einem N-Atom und einem S-Atom im Ring und mit einem C₁-Alkyl-Substituenten bedeuten und
(ii) X-Y als Gruppe der Formel -CH₂-CH-OP ausgenommen ist, wenn R¹ ein H-Atom oder eine C₁₋₄-Alkylgruppe und R² einen mono-cyclischen Heteroaromaten mit einem N-Atom und einem S-Atom im Ring und mit einem C₁-Alkyl-Substituenten bedeuten.

6. Epothilonderivat nach einem der vorstehenden Ansprüche, **dadurch gekennzeichnet, daß** R¹, R⁴, R⁵ und R⁶ ein H-Atom oder eine C₁- bis C₆-Alkylgruppe, vorzugsweise eine C₁₋₆-Alkylgruppe sind.

7. Epothilonderivat nach einem der Ansprüche 4 bis 6, **dadurch gekennzeichnet, daß** die Substituenten des monocyclischen Aromaten und/oder Heteroaromaten C₁₋₆-Alkyl- bzw. C₂₋₆-Alkenyl bzw. C₂₋₆-Alkinylgruppen, vorzugsweise C₁₋₄-Alkyl- bzw. C₂₋₄-Alkenyl bzw. C₂₋₄-Alkinylgruppen sind und die Halogenatome Fluor-, Chlor-, Brom- oder Jodatome sind.

8. Epothilonderivat nach einem der Ansprüche 4 bis 7, **dadurch gekennzeichnet, daß** der Aromat bzw. Heteroaromat 1, 2 oder 3 Substituenten und der Heteroaromat 1, 2 oder mehr und insbesondere 1, 2, 3, oder 4 Heteroatome aufweist.

9. Verfahren zur Herstellung einer Verbindung der Formel (3), **dadurch *gekennzeichnet,* daß** eine Verbindung der Formel (2) mit einer Verbindung der Formel HC[B(OR)₂]₃ ggf. mit Hilfe einer Base umgesetzt wird, wobei die Reste wie in einem der vorstehenden Ansprüche definiert sind und R wie R¹ definiert aber von R¹ unabhängig ist.

10. Verfahren zur Herstellung einer Verbindung der Formel (4), **dadurch *gekennzeichnet,* daß** eine Verbindung der Formel (3) mit N-Iod- oder N-Bromsuccinimid umgesetzt wird und die Reste wie in einem der vorstehenden Ansprüche definiert sind.

11. Verfahren zur Herstellung einer Verbindung der Formel (5), **dadurch *gekennzeichnet,* daß** eine Verbindung der Formel (3) durch eine Suzuki-Kopplung mit einer Verbindung der Formel R²-Z umgesetzt wird, wobei R² wie in einem der vorstehenden Ansprüche definiert ist und Z ein Halogenatom oder eine Gruppe der Formel -OSO₂CF₃, -CH=CHI, -CH=CHOSO₂CF₃ sein kann.

12. Verfahren zur Herstellung einer Verbindung der Formel (5), **dadurch *gekennzeichnet,* daß** eine Verbindung der Formel (4) durch eine Stille-Kupplung mit R²-SnR³₃ umgesetzt wird, wobei R² wie in einem der vorstehenden Ansprüche definiert ist und R³ eine C₁₋₆-Alkylgruppe, vorzugsweise eine C₁₋₄-Alkylgruppe, besonders bevorzugt eine Methyl-, Ethyl-, Propyl- oder Butylgruppe ist.

13. Verfahren zur Herstellung einer Verbindung der Formel (6), **dadurch *gekennzeichnet,* daß** von einer Verbindung der Formel (5) die Schutzgruppe entfernt wird.

14. Verfahren zur Herstellung einer Verbindung der Formel (6), **dadurch *gekennzeichnet,* daß** es die Verfahrensstufen umfaßt, die in den Ansprüchen 9, 10, 11 oder 12 und 13 offenbart sind, wobei die Reste wie in den vorstehenden Ansprüchen definiert sind.

15. Arzneimittel, das mindestens eine der in den Ansprüchen 1 bis 8 beschriebenen Verbindungen und gegebenenfalls übliche Träger, Verdünnungsmittel und/oder Adjuvantien enthält.

16. Arzneimittel nach Anspruch 15, **dadurch gekennzeichnet, daß** es sich um ein Cytostaticum handelt.

17. Pflanzenschutzmittel in der Landwirtschaft und/oder Forstwirtschaft und/oder im Gartenbau, das mindestens eine der in den Ansprüchen 1 bis 8 beschriebenen Verbindungen und gegebenenfalls übliche Träger, Verdünnungsmittel und/oder Adjuvantien enthält.

## Claims

1. Epothilone derivative of formula (2) 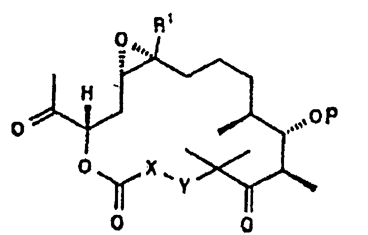
wherein R¹ is a hydrogen atom or a C₁₋₈-alkyl group, X-Y is a group of formula -CH₂CH-OP or -CH=CH-, and
P is a protective group, wherein
X-Y is excluded as group of formula -CH₂CH-OP if R¹ means a hydrogen atom or a C₁₋₄-alkyl group.

2. Epothilone derivative of formula (3) 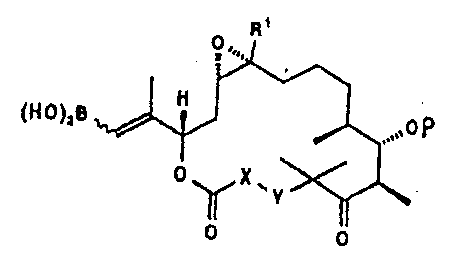 wherein the residues are as defined in claim 1.

3. Epothilone derivative of formula (4) 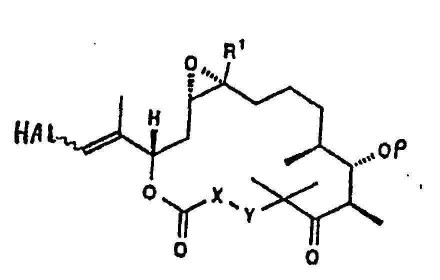 wherein the residues R¹, X-Y and P are defined as in claim 1, and Hal is a halogen atom such as Br or I.

4. Epothilone derivative of formula (5) 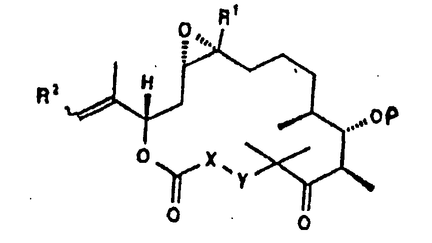 wherein the residues R¹, X-Y and P are defined as in claim 1, and R² is a monocyclic aromatic which can be substituted by a halogen atoms and/or OR⁴- and/or NR⁵R⁶- and/or alkyl, alkenyl and/or alkinyl groups in ortho- and/or meta- and/or para-position, or a monocyclic 5- or 6-membered hetero aromatic, which can be provided with one or several O- and/or N- and/or S-atoms in the ring and/or which can be provided with OR⁴- and/or NR⁵R⁶- and/or alkyl, alkenyl and/or alkinyl groups as substituents, wherein the residues R⁴, R⁵ and R⁶ independently are defined as R¹ in claim 1, but are independent of R¹, wherein
(i) X-Y is excluded as group of formula -CH=CH- if R¹ is a hydrogen atom or a C₁₋₄-alkyl group and R² is a monocyclic hetero aromatic having a N atom and a S atom in its ring and a C₁-alkyl substituent and
(ii) X-Y is excluded as group of formula -CH₂-CH-OP if R¹ is a hydrogen atom or a C₁₋₄-alkyl group and R² is a monocyclic hetero aromatic having a N atom and a S atom in its ring and a C₁-alkyl substituent.

5. Epothilone derivative of formula (6) 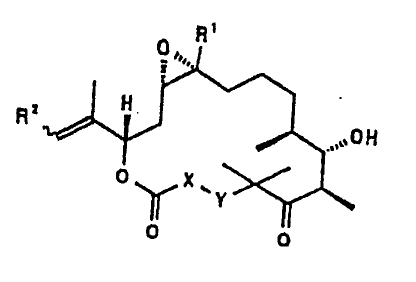 wherein the residues are defined as in claim 4 and, if X-Y means a group of formula -CH₂CH-OP, the protective group P has been removed, wherein
(i) X-Y is excluded as group of formula -CH=CH- if R¹ is a hydrogen atom or a C₁₋₄-alkyl group and R² is a monocyclic hetero aromatic having a N atom and a S atom in its ring and a C₁-alkyl substituent and
(ii) X-Y is excluded as group of formula -CH₂-CH-OP if R¹ is a hydrogen atom or a C₁₋₄-alkyl group and R² is a monocyclic hetero aromatic having a N atom and a S atom in its ring and a C₁-alkyl substituent.

6. Epothilone derivative according to any of the preceding claims, **characterized in that** R¹, R⁴, R⁵ and R⁶ are a hydrogen atom or a C₁₋₆-alkyl group, especially a C₁₋₆-alkyl group.

7. Epothilone derivative according to any of claims 4 to 6, **characterized in that** the substituents of the monocyclic aromatic and/or hetero aromatic are C₁₋₆-alkyl, C₂₋₆-alkenyl and C₂₋₆-alkinyl groups, respectively, especially C₁₋₄-alkyl, C₂₋₄-alkenyl and C₂₋₄-alkinyl groups, respectively and the halogen atoms fluoro, chloro, bromo or iodo atoms.

8. Epothilone derivatives according to any of claims 4 to 7, **characterized in that** the aromatic and hetero aromatic, respectively, is provided with 1, 2 or 3 substituents and the hetero aromatic is provided with 1, 2 or more and especially 1, 2, 3, or 4 hetero atoms.

9. Process for the production of a compound of formula (3), **characterized in that** a compound of formula (2) is reacted with the compound of formula HC[B(OR)₂]₃ if wanted in the presence of a base, wherein the residues are defined as in any of the preceding claims and R is defined as R¹, but is independent of R¹.

10. Process for the production of a compound of formula (4), **characterized in that** a compound of formula (3) is reacted with N-iodo- or N-bromo succinimide and that the residues are defined as in any of the preceding claims.

11. Process for the production of a compound of formula (5), **characterized in that** a compound of formula (3) is reacted by a Suzuki coupling with a compound of formula R²-Z , wherein R² is defined as in any of the preceding claims and Z can be a halogen atom or a group of formula -OSO₂CF₃, -CH=CHI, -CH=CHOSO₂CF₃.

12. Process for the production of a compound of formula (5), **characterized in that** a compound of formula (4) is reacted by a Stille coupling (Stille Kupplung) with R²-SNR³₃, wherein R² is defined as in any of the preceding claims and R³ is a C₁₋₆-alkyl group, especially a C₁₋₄-alkyl group, preferably a methyl, ethyl, propyl or butyl group.

13. Process for the production of a compound of formula (6), **characterized in that** the protective group is removed from a compound of formula (5).

14. Process for the production of a compound of formula (6), **characterized in that** it comprises the process steps as disclosed in claims 9, 10, 11 or 12 and 13, wherein the residues are defined as in the preceding claims.

15. Therapeutical agent, containing at least one of the compounds described in claims 1 to 8 and optionally usual carriers, diluents and/or auxiliary agents.

16. Therapeutical agent according to claim 15, **characterized in that** it is a cytostaticum.

17. Plant protecting agent in agriculture and/or forest culture and/or horticulture, containing at least one compound described in claims 1 to 8 and optionally usual carriers, diluents and/or auxiliary agents.

## Revendications

1. Dérivé d'épothilone de formule (2) 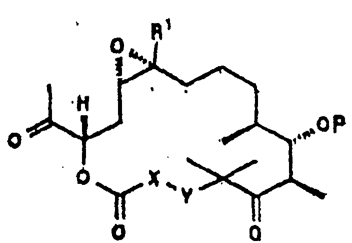 dans lequel R¹ représente un atome d'hydrogène ou un groupe alkyle en C₁₋₈, X-Y représente un groupe de formule -CH₂CH-OP ou -CH=CH-, et P un groupe de protection, X-Y ne représentant pas un groupe de formule -CH₂CH-OP lorsque R¹ représente un atome d'hydrogène ou un groupe alkyle en C₁₋₄.

2. Dérivé d'épothilone de formule (3) 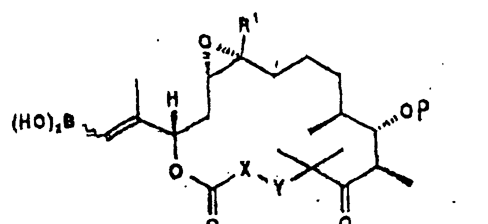 dans laquelle les symboles sont définis comme dans la revendication 1.

3. Dérivé d'épothilone de formule (4) 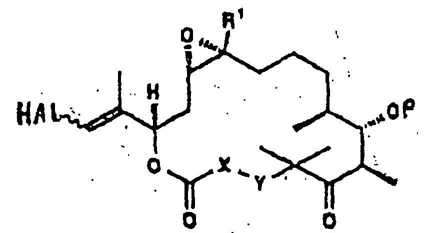 dans laquelle les symboles R¹, X-Y et P sont définis comme dans la revendication 1, et Hal représente un atome d'halogène tel que Br ou I.

4. Dérivé d'épothilone de formule (5) 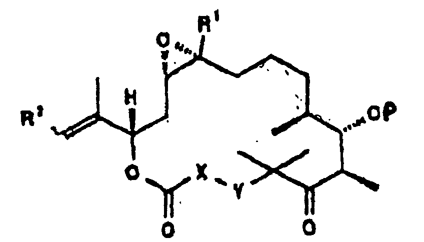 dans laquelle les symboles R¹, X-Y et P sont définis comme dans la revendication 1, R² représente un résidu aromatique monocyclique portant éventuellement un ou plusieurs substituants halogéno et/ou -OR⁴ et/ou -NR⁵R⁶ et/ou alkyle, alcényle et/ou alcynyle en position ortho et/ou méta et/ou para, ou un résidu monocyclique hétéroaromatique à 5 ou 6 chaînons, qui peut comporter, dans son cycle, un ou plusieurs atomes d'oxygène et/ou d'azote et/ou de soufre, et/ou qui peut porter un ou plusieurs résidus -OR⁴ et/ou -NR⁵R⁶ et/ou alkyle, alcényle et/ou alcynyle, les résidus R⁴, R⁵ et R⁶ ayant indépendamment les uns des autres la signification de R¹ dans la revendication 1, mais indépendante de celle de R¹,
(i) X-Y ne représentant pas un groupe -CH=CH- lorsque R¹ est un atome d'hydrogène ou un groupe alkyle en C₁₋₄ et R² représente un noyau monocyclique hétéroaromatique comportant, dans son cycle, un atome d'azote et un atome de soufre et portant un substituant alkyle en C₁, et
(ii) X-Y ne représentant pas un groupe de formule -CH₂-CH-OP lorsque R¹ représente un atome d'hydrogène ou un groupe alkyle en C₁₋₄ et R² représente un groupe monocyclique hétéroaromatique comportant un atome d'azote et un atome de soufre et portant un substituant alkyle en C₁.

5. Dérivé d'épothilone de formule (6) 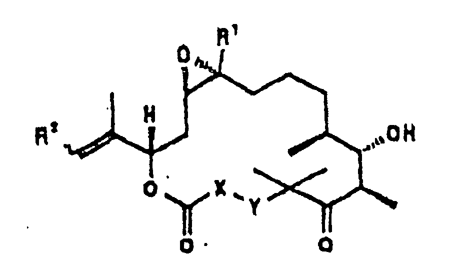 dans laquelle les symboles sont définis comme dans la revendication 4, et dans laquelle, lorsque X-Y représente un groupe de formule -CH₂-CH-OP, le groupe de protection P de celui-ci est éliminé,
(i) X-Y ne représentant pas un groupe -CH=CH- lorsque R¹ est un atome d'hydrogène ou un groupe alkyle en C₁₋₄ et R² représente un noyau monocyclique hétéroaromatique comportant, dans son cycle, un atome d'azote et un atome de soufre et portant un substituant alkyle en C₁, et
(ii) X-Y ne représentant pas un groupe de formule -CH₂-CH-OP lorsque R¹ représente un atome d'hydrogène ou un groupe alkyle en C₁₋₄ et R² représente un groupe monocyclique hétéroaromatique comportant un atome d'azote et un atome de soufre et portant un substituant alkyle en C₁.

6. Dérivé d'épothilone selon l'une des revendications précédentes, **caractérisé par le fait que** R¹, R⁴, R⁵ et R⁶ représentent un atome d'hydrogène ou un groupe alkyle en C₁₋₆, de préférence un groupe alkyle en C₁₋₆.

7. Dérivé d'épothilone selon l'une des revendications 4 à 6, **caractérisé par le fait que** les substituants sur les groupes monocycliques aromatique et/ou hétéroaromatique sont des groupes alkyle en C₁₋₆ ou alcényle en C₂₋₆ ou alcynyle en C₂₋₆, de préférence des groupes alkyle en C₁₋₄ ou alcényle en C₂₋₄ ou alcynyle en C₂₋₄, et les atomes d'halogène sont des atomes de fluor, de chlore, de brome et d'iode.

8. Dérivé d'épothilone selon l'une des revendications 4 à 7, **caractérisé par le fait que** le groupe aromatique ou hétéroaromatique porte 1, 2 ou 3 substituants et que le groupe hétéroaromatique porte 1 ou 2 substituants ou plus, et en particulier 1, 2, 3 ou 4 hétéroatomes.

9. Procédé de préparation d'un composé de formule (3) **caractérisé par le fait que** l'on fait réagir un composé de formule (2) avec un composé de formule HC[B(OR)₂]₃ éventuellement en présence d'une base, les différents symboles étant définis comme dans l'une des revendications précédentes et R étant défini comme R¹ tout en étant indépendant de celui-ci.

10. Procédé de préparation d'un composé de formule (4), **caractérisé par le fait que** l'on fait réagir un composé de formule (3) avec du N-iodo-succinimide ou du N-bromosuccinimide, les différents symboles ayant la signification indiquée dans l'une des revendications précédentes.

11. Procédé de préparation d'un composé de formule (5), **caractérisé par le fait que** l'on soumet un composé de formule (3) et un composé de formule R²-Z à une réaction de couplage Suzuki, R² ayant la signification indiquée dans l'une des revendications précédentes et Z représentant un atome d'halogène ou un groupe de formule -OSO₂CF₃, -CH=CHI-, -CH=CHOSO₂CF₃.

12. Procédé de préparation d'un composé de formule (5) **caractérisé par le fait que** l'on soumet un composé de formule (4) à un couplage Stille avec un composé de formule R²-SnR³₃, où R² est défini de la manière indiquée dans l'une des revendications précédentes et R³ représente un groupe alkyle en C₁₋₆, de préférence alkyle en C₁₋₄, et en particulier un groupe méthyle, éthyle, propyle ou butyle.

13. Procédé de préparation d'un composé de formule (6), **caractérisé par le fait que** l'on élimine le groupe de protection du composé de formule (5).

14. Procédé de préparation d'un composé de formule (6), **caractérisé par le fait qu'**il comprend une étape de procédé telle que celles divulguées dans les revendications 9, 10, 11 ou 12 et 13, les différents symboles ayant la signification indiquée dans les revendications précédentes.

15. Médicament contenant au moins un des composés décrits dans l'une des revendications 1 à 8 et éventuellement des excipients, diluants et/ou adjuvants usuels.

16. Médicament selon la revendication 15, **caractérisé par le fait qu'**il s'agit d'un médicament cytostatique.

17. Composition phytosanitaire pour l'agriculture et/ou l'exploitation forestière et/ou le jardinage, contenant au moins un des composés décrits dans l'une des revendications 1 à 8 et éventuellement des excipients, diluants et/ou adjuvants usuels.
